# EUROPEAN PATENT APPLICATION

(11) **EP 3 915 470 A1**
(43) Date of publication of application: **01.12.2021**
(21) Application number: 20020250.5
(22) Date of filing: 26.05.2020
(51) Int. Cl.: A61B 5/021, A61N 1/36, A61N 1/05

(54) **NEUROMODULATION AND/OR NEUROSTIMULATION SYSTEM**

(71) Applicant: ONWARD Medical B.V., 5656 AE Eindhoven (NL)
(72) Inventor: CABAN, Miroslav, 5656AE Eindhoven (NL); VON ZITZEWITZ, Joachim, 5656AE Eindhoven (NL)
(74) Representative: Wende, Christian Werner

(57) **Abstract**

A neuromodulation and/or neurostimulation system (10, 110) comprising at least the following components:
- at least one sensing unit (12, 112), wherein the sensing unit (12, 112) is configured to provide a sensor signal correlating with a physiological value, which describes neurological function or dysfunction, preferably autonomic function or dysfunction of a patient;
- at least one control unit (14, 114),
- at least one stimulation unit (16, 116),
- at least one Central Nervous System (CNS) stimulation module for providing CNS stimulation (18), and/or at least one Peripheral Nervous System (PNS) stimulation module for providing PNS stimulation (20, 120);
wherein the control unit (14, 114) is configured to detect a dysfunction of the neurological function, preferably autonomic function of the patient based on the sensor signal and to trigger neuromodulation for stabilization and/or treatment of the neurological function, preferably autonomic function of the patient.

The invention further relates to a method for providing neuromodulation and/or neurostimulation and the use of a neuromodulation system in a method for the treatment of a patient.

## Description

The present invention relates to a system for neuromodulation, especially neurostimulation, for the treatment of a subject, in the field of improving for example after disorders or injuries of the central nervous system (CNS), such as the spinal cord.

The present invention further relates to a method for providing neuromodulation and/or neurostimulation and the use of a system for neuromodulation, especially neurostimulation in a method for the treatment of a patient after disorders or injuries of CNS, such as the spinal cord.

The spinal cord is an integral part of the CNS. Spinal cord injury (SCI), but also other disorders or injuries (e.g. stroke, brain injury, cerebral palsy, multiple sclerosis, autonomic failure, autonomic neuropathy and/or cancer of the neurological tissue) often results in motor deficits. For instance, SCI interrupts the communication between the spinal cord and supraspinal centres, depriving these sensorimotor circuits from the excitatory and modulatory drives necessary to produce movement.

Normal autonomic function is critically dependent on the interaction between supraspinal centers and spinal autonomic components. Descending neuronal projections from the brainstem and hypothalamus regulate sympathetic and parasympathetic activities at the spinal cord level. Traumatic SCI (and/or disorders such as stroke, multiple sclerosis, autonomic failure, autonomic neuropathy and/or cancer of the neurological tissue) can interrupt connections between higher centers and the spinal cord, resulting not only in somato-motor and sensory deficits but also in autonomic dysfunctions. In particular, SCI and/or the above-mentioned disorders often result in disconnection of some, most, or all descending pathways that carry signals responsible for regulating arterial blood pressure, heart rate, respiratory function, but also sexual function, thermoregulation and/or gut and/or bladder and/or bowel function, production of body fluids (saliva, sweat, tears), balance of water and electrolytes (sodium and calcium), metabolism, digestion.

From a historical perspective, autonomic dysfunctions have not been the focus of basic or clinical research when compared to the amount of attention that "curing paralysis" has received. However, in recent decades autonomic disorders after SCI have drawn more investigations as researchers and clinicians began to pronounce their clinical priority. Autonomic dysfunction following SCI is a potentially life-threatening condition that leads to blood pressure instability and ensuing chronic dysfunction of the heart and vasculature. Most SCI patients experience multiple drastic, blood pressure fluctuations daily, and rank this as a top healthcare priority.

Individuals with severe SCI at the level of T6 (spinal segment) or above often suffer from autonomic dysfunction and/or dysregulated cardiovascular control (to a lesser extend also individuals with SCI at the level of T8-T10 (spinal segment)). Usually, the higher the level of the spinal cord injury the more severe is the hypertension in autonomic dysfunction.

Without further specification 'blood pressure' often may refer to the pressure in arteries of the systemic circulation. Blood pressure may usually be expressed in terms of systolic blood pressure, and/or over diastolic blood pressure and/or over mean arterial blood pressure, i.e. an average blood pressure in an individual during a single cardiac cycle, and may be measured in millimeters of mercury (mmHg, above the surrounding atmospheric pressure).

Blood pressure monitoring may comprise monitoring a parameter value such as diastolic blood pressure, systolic blood pressure, diastolic blood pressure and a systolic blood pressure, mean arterial pressure, or the like.

Both, dangerous decreases (i.e. hypotension) or elevations (i.e. hypertension) of blood pressure, in particular arterial blood pressure, may occur as a consequence of SCI, as the spinal cord neurons responsible for blood pressure control no longer have the capacity to maintain blood pressure at a normal physiological level. Hypotension may for instance lead to dizziness, disorientation, reduction in cognitive functioning, loss of consciousness and a predisposition to strokes and heart attacks. Hypertension may for instance lead to heart attacks, strokes, and sub-clinical vascular consequences.

Neuromodulation, in particular neurostimulation by means of neuromodulation system/ neurostimulation system is a well-established neuromodulatory/neurostimulatory therapy not only for restoring locomotion/motoric function after spinal cord injury or central nervous system diseases, but also for treating inter alia pain and/or restoring autonomic function.

Known stimulation systems for treating patients after spinal cord injury (e.g. to restore motor function and/or blood pressure) mainly use either CNS stimulation, especially Epidural Electrical Stimulation (EES), or Peripheral Nerve System (PNS) Stimulation, especially Functional Electrical Stimulation (FES).

EES is known to restore motor control in animal and human models and has more particularly been shown to restore locomotion after spinal cord injury by artificially activating the neural networks responsible for locomotion below the spinal cord lesion ***(***Capogrosso et al., A Computational Model for Epidural Electrical Stimulation of Spinal Sensorimotor Circuits, Journal of Neuroscience 4 December 2013, 33 (49) 19326-19340*,* Courtine et al., Transformation of nonfunctional spinal circuits into functional states after the loss of brain input, Nat Neurosci. 2009 Oct; 12(10): 1333-1342*.* Moraud et al, Mechanisms Underlying the Neuromodulation of Spinal Circuits for Correcting Gait and Balance Deficits after Spinal Cord Injury, Neuron Volume 89, Issue 4, p814-828, 17 February 2016*).* EES does not directly stimulate motor-neurons but the afferent sensory neurons prior to entering into the spinal cord. In this way, the spinal networks responsible for locomotion are recruited indirectly via those afferents, restoring globally the locomotion movement by activating the required muscle synergies. The produced movement is functional; however, due to relatively poor selectivity (network activation instead of selective targeting of key muscles) the controllability is low and the imprecisions hinder fluidity and full functionality in the potential space of the movement. EES may also be applied for restoring autonomic function after SCI (WO2018148844A1).

PNS stimulation systems used to date in the clinic are known as FES that provides electrical stimulation to target muscles with surface electrodes, either directly through stimulation of their motorfibers (neuro-muscular stimulation), or through a limited set reflexes (practically limited to the withdrawal reflex) or by transcutaneously stimulating the peripheral nerves. The resulting muscle fatigue has rendered FES unsuitable for use in daily life. Furthermore, successes have remained limited through cumbersome setups when using surface muscle stimulation, unmet needs in terms of selectivity (when using transcutaneous nerve stimulation) and a lack of stability (impossible to reproduce exact electrode placement on a daily basis when stimulating muscles, moving electrodes due to clothes, sweating).

EP 2 868 343 A1 discloses a system to deliver adaptive electrical spinal cord stimulation (in particular EES) to facilitate and restore locomotion after neuromotor impairment. Inter alia, a closed-loop system for real-time control of EES is disclosed, comprising means for applying to a subject neuromodulation with adjustable stimulation parameters, means being operatively connected with a real-time monitoring component comprising sensors continuously acquiring feedback signals from subject, signals providing features of motion of a subject, system being operatively connected with a signal processing device receiving feedback signals and operating real-time automatic control algorithms, signal processing device being operatively connected with means and providing means with new stimulation parameters, with minimum delay. This known system improves consistency of walking in a subject with a neuromotor impairment. Reference is also made to Wenger et al., Closed-loop neuromodulation of spinal sensorimotor circuits controls refined locomotion after complete spinal cord injury, in Science Translational Medicine, vol. 6, num. 255, 2014.

WO2018/148844A1 discloses a device and algorithm for controlling an autonomic function in an individual, by providing CNS stimulation. In particular, a controller device is disclosed that utilizes physiological measurements (such as blood pressure) to regulate spinal cord electrical stimulation to stabilize blood pressure. A control interface and algorithm for controlling an autonomic function in a subject. In particular, an algorithm that utilizes physiological measurements is disclosed (such as blood pressure) to regulate spinal cord electrical stimulation to stabilize blood pressure. The neuronal structures involved may be located within the T1 to S5 segments of the spinal cord, for example. Stimulation may be configured to control a particular function by selecting electrodes and/or the nature of the stimulation.

US2011/0202107A1 describes an electric stimulation apparatus for treating hypotension of patients with spinal cord injury and a method for treating hypotension. An electric stimulation apparatus of the present invention comprises: a blood pressure measuring means for continuously measuring a blood pressure of a subject; an electric current application means comprising an electrode for intermittently applying an electric current to skin of the subject; and a control means for controlling the electric current application means so as to maintain the blood pressure at a predetermined target blood pressure value by activating the electric current application means when the subject blood pressure is equal to or less than the target blood pressure value. The electrode can be positioned within inguinal, femoral, lumbar and lower abdominal regions of the subject.

It is an object of the present invention to provide an improved system and/or method to control impaired neurological function, especially impaired autonomic function, by limiting side effects when applying neuromodulation to a patient suffering from autonomic dysfunction.

This object is solved according to the present invention with a system for neuromodulation and/or neurostimulation with the features of claim 1. Accordingly, a neuromodulation and/or neurostimulation system comprising at least the following components:
- at least one sensing unit wherein the sensing unit is configured to provide a sensor signal correlating with a physiological value, which describes neurological function or dysfunction, preferably autonomic function or dysfunction of a patient;
- at least one control unit,
- at least one stimulation unit,
- at least one Central Nervous System (CNS) stimulation module for providing CNS stimulation, and/or at least one Peripheral Nervous System (PNS) stimulation module for providing PNS stimulation;
- wherein the control unit is configured to detect a dysfunction of the neurological function, preferably autonomic function of the patient based on the sensor signal and to trigger neuromodulation for stabilization and/or treatment of the neurological function, preferably autonomic function of the patient.

The invention is based on the basic idea that a neurostimulation system is provided which specifically targets and modulates neurons and/or smooth muscles and/or muscles responsible for and/or involved in autonomous control in a way that the system enables precise and optimized control over autonomous control after SCI. The use of a hardware system comprising at least one sensing unit, control unit, stimulation unit and CNS stimulation module and/or a PNS stimulation module enables optimized neurostimulation by sensing physiological parameters of neurological function, especially autonomic function (or dysfunction) of a patient, defining and/or using a target value of neurological function, especially autonomic function and identification of optimal stimulation parameters for restoring neurological function, especially autonomous function to minimize the difference between the sensed physiological parameters of neurological function, especially autonomic function (or dysfunction) and the respective target value. A central role may play the control unit, comparing the physiological status of the patient with the respective target value, and calculating optimal stimulation parameters, such as electrode configuration, frequency, amplitude and/or pulse width, for equaling the physiological parameters to the target value, and at the same time minimizing effects on other functions and/or responses to the stimulation, e.g. muscle function. A further central role may play the stimulation unit, providing neuromodulation via the CNS stimulation module and/or PNS stimulation module, in order to provide optimal stimulation and/or minimize side effects of stimulation. So it is possible to improve a neuromodulation system, e.g. in the field of improving recovery after neurological disorders like SCI, especially in that neuromodulation and/or neurostimulation can be provided in almost any environment and in daily life, adapted to the patient's needs and providing the needed assistance in training and daily life for the patient, also adjusted to the progress of the rehabilitation of the patient. One further advantage of the system according to the present invention is, that the system could be combined and/or connected and/or included in a system for restoring motoric function after spinal cord injury and/or disorders (e.g. stroke, cerebral palsy, brain injury, multiple sclerosis, autonomic failure, autonomic neuropathy and/or cancer of the neurological tissue which impair operation of descending pathways that normally facilitate control of motoric function).

Autonomic function may be or may comprise blood pressure (systolic blood pressure, diastolic blood pressure, mean arterial blood pressure), heart rate, respiratory function, but also sexual function, thermoregulation and/or gut and/or bladder and/or bowel function, production of body fluids (saliva, sweat, tears), balance of water and electrolytes (sodium and calcium), metabolism, digestion.

Furthermore, neurological functions and/or dysfunctions that may be treated and/or stabilized with the neuromodulation system may be or may comprise spasticity, clonus and/or pain.

In general, the system may be used to continuously control and/or maintain neurological function, especially autonomic function of a patient, and/or prevent critical situations, such as attacks of autonomic dysreflexia.

The sensing unit may be or may comprise at least one sensor. In particular, the sensing unit may be or may comprise at least one of a blood pressure sensor, a pulse oximeter, a heart-rate sensor, a breathing rate sensor, a body temperature sensor, a pressure sensor (for instance for sensing bladder internal pressure), a pH sensor, a barometer, an inertial measurement unit, an accelerometer, a gyroscope, a chemical concentration sensor (glucose, water, sodium, calcium), a hormone sensor, a metabolites sensor, a sweat sensor, a gastrointestinal movement sensor, a hydration sensor, an electrolyte sensor, an arterial stiffness sensor, a urine concentration sensor, a urine volume sensor, a bladder volume sensor, a conductivity sensor, a capacitance sensor, a strain gauge sensor, a genital blood flow sensor. Further, such a sensing unit may enable that the system is a closed-loop system (feedback mechanisms based on sensed data).

Further, it is generally possible that at least two sensors may from a sensor network. Thus, for instance, a blood pressure sensor and a pulse oximeter and/or a heart-rate sensor and/or a body temperature sensor may be combined for sensing patient data. This may enable an optimal monitoring of more than one physiological status and/or performance, based on which an optimal therapy may be provided by the system.

Furthermore, it is conceivable that at least one of the components of the neuromodulation and/or neurostimulation system may be implantable.

The at least one sensor unit and/or sensor may be invasive or non-invasive. In other words, the at least one sensor may be at least partially implantable and/or implanted. Alternatively, the at least one sensor may be not implanted and/or not implantable. Further, it is possible that the at least one sensor is a digital or analog sensor system. A sensor network may generally comprise both at least one at least partially implanted and/or implantable sensor and at least one non-implantable and/or non-implanted sensor.

A blood pressure sensor may measure and/or monitor systolic and/or diastolic and/or mean arterial pressure (and/or also spinal cord perfusion pressure) of the patient.

An implanted and/or implantable sensor and/or a non-implantable and/or non-implanted sensor may be, but is not limited to, an upper arm blood pressure monitor system or a wrist blood pressure monitor system or a finger blood pressure monitor system. The sensor may measure and/or monitor a blood pressure signal indicative for a blood pressure measurement. In general, the at least one sensor may provide continuous monitoring of blood pressure and/or sporadic monitoring of blood pressure and/or measuring or monitoring blood pressure in preset time intervals.

Furthermore, it is conceivable that the sensor may be or may comprise at least one of an electrocardiogram (ECG), an electroencephalogram (EEG), or an electromyogram (EMG), a patch clamp, a voltage clamp, an extracellular single-unit recorder, a recorder of local field potentials. An ECG may sense the cardiac rhythm (heart rate) of a patient. The cardiac rhythm may then be related to a status of cardiac output, e.g. a status with good cardiac output, a status of medium cardiac output, or a status of bad cardiac output. The ECG may comprise sensors placed on the skin of the patient, which enables immediate, non-invasive and low-cost sensing of physiological parameters (cardiac rhythm, heart rate) of the patient.

An EEG may sense summed electrical activity of the brain by recording the voltage fluctuations on the head surface. The EEG may comprise sensors placed on the skin/head of the patient, which also enables immediate, non-invasive and low-cost sensing of physiological parameters (electrical brain activity) of the patient. Sensing patient physiological parameters by electroencephalography may enable to correct noisy ECG and/or other signals (obtained by blood pressure sensing, etc.). Physiological parameters sensed by the sensing unit, e.g. blood pressure or cardiac rhythm (by ECG) may be not reliable due to unexpected noise that distort the signals. It may be possible that the system, in particular the control unit corrects such artifacts, e.g. ECG artifacts, by using the EEG signals.

Further, it is conceivable that the sensing unit is configured and arranged and/or the control unit is configured and arranged for optimizing stimulation parameters for stabilization and/or treatment of the neurological function, preferably autonomic function of the patient. In particular, stimulation parameters may be or may comprise electrode configuration, frequency, amplitude and/or pulse with of at least one pulse train. Thus, the sensing unit that can directly and/or indirectly measure the effect of the provided stimulation on the desired neurological system, especially autonomic system, and/or the control unit that can perform an approximate mapping may enable automatic and/or semi-automatic optimization of the stimulation parameters.

Further, it is possible that the sensing unit may be or may comprise at least one trigger, e.g. a manual trigger to be triggered by the patient and providing a signal describing dysfunction of the autonomic function of the patient. The trigger may for example be or may comprise an emergency button. For instance, when a patient recognizes feelings or conditions including but not limited to dizziness, disorientation, nausea, reduction in cognitive functioning, beginning loss of consciousness, sweating, shortness of breath, blue lips, no sphincter relaxation, loss of urine, loss of feces and/or spasticity, triggering the trigger may enable to restore autonomic function and/or at least partially remedy the above-mentioned symptoms. In particular, this may be advantageous when the system is used as an open-loop system without adaptation of stimulation parameters to feedback data and/or when the control mechanisms of the system, when used as a closed-loop system, fail.

The stimulation unit may comprise a pulse generator, e.g. an implantable pulse generator, and/or one electrode and/or a plurality of electrodes. Subsets of electrodes may be defined as any value x out of the range 0 to n, n being the number of electrodes available. The plurality of electrodes may be arranged in an array, e.g. in an electrode array on a stimulation lead/paddle. Such a stimulation lead may be a lead paddle or a lead wire or any kind of lead or carrier(s) having at least one lead or carrying at least a partial number of the electrodes.

The CNS stimulation module and/or the PNS stimulation module may comprise at least one electrode. It may be generally possible that the CNS stimulation module and/or the PNS stimulation module may comprise a plurality of electrodes. Also, it may be possible that the CNS stimulation module and/or the PNS stimulation module each comprise a pulse generator, e.g. an implantable pulse generator. Also, it may be possible that the stimulation unit comprises the CNS stimulation module and/or the PNS stimulation module.

In one embodiment, the CNS stimulation module and/or the PNS stimulation module may be comprised in the sensing unit.

It is generally possible that the CNS stimulation module is or comprises an epidural stimulation module capable to provide epidural stimulation and/or a trans-cutaneous stimulation module capable to provide transcutaneous stimulation and/or a subdural stimulation module capable to provide subdural stimulation and/or an intracortical stimulation module capable to provide intracortical stimulation and/or an intraspinal stimulation module capable to provide intraspinal stimulation. Depending on the physiological constitution of the patient and the injury or disorder of the patient, most suitable CNS stimulation, and thus the most suitable CNS stimulation module may be provided. The epidural stimulation module may comprise at least one electrode, which can be invasive and implanted in the spinal channel (epidurally). Similarly, the subdural stimulation module may comprise at least one electrode, which can be invasive and implanted subdurally. The epidural electrode(s) and/or subdural electrode(s) can be implanted in the vertebral channel through minimally invasive or invasive surgical techniques. However, the system is not limited to such an application. An intracortical stimulation module may comprise at least one electrode, can be invasive and implanted intracortically. A transcutaneous stimulation module can comprise at least one electrode, which can be non-invasive and may be placed on the neck and/or back of the patient treated with the system, along the spine. Generally speaking, stimulation electrode arrays can also be non-invasive, e.g. by administering and providing transcutaneous stimulation to the spinal cord and/or other parts of the nervous system.

The PNS stimulation module may comprise at least one electrode, which can be invasive and implanted and/or implantable and/or noninvasive (the latter means that stimulation is provided transcutaneously). PNS stimulation may be applied to the limbs and/or the abdomen of a patient. The electrodes may be, for instance, placed on the abdomen and/or limbs (legs, arms) of a patient treated with the system. In particular, the electrodes (implanted or non-implanted) may be placed closely to large vessels of the patient.

The PNS stimulation module may be a Functional Electrical Stimulation (FES) module capable to provide electrical stimulation to peripheral nerves and/or muscles.

In particular, PNS stimulation may be applied to directly evoke vasoconstriction and/or vasodilatation by contraction/dilatation of smooth muscles of the vessels and/or to evoke blood pressure alterations by stimulation of skeletal muscles.

Moreover, the CNS stimulation module may be at least partially implantable or at least partially implanted and/or that the PNS stimulation module may be at least partially implantable or at least partially implanted. This means that at least the electrodes and/or the electrode array and/or the pulse generator of the CNS stimulation module and/or the PNS stimulation module may be implantable. The implantable pulse generator(s) and/or electrodes (e.g. in the form of an electrode paddle with an array of electrodes) can be implanted minimally invasive or invasive. This has the advantage of improved ability of the patient treated with the system to function and participate in activities of daily living.

In a possible embodiment, the components of the neuromodulation and/or neurostimulation system may form a closed-loop system. This has the advantage that the stimulation provided by the system may be adapted to specific needs and/or condition of the patient, the stimulation being adjusted according to feedback data of the patient, provided via the sensing unit. Alternatively, the components of the neuromodulation and/or neurostimulation system may form an open-loop system. In particular, open-loop may be understood as delivery of pre-programmed spatiotemporal stimulation or pre-programmed spatiotemporal stimulation patterns with spatial and temporal components. With such an open-loop system open-loop phasic stimulation may be provided. In contrast to closed-loop systems, open-loop may be understood such that neuromodulation or neurostimulation is provided, but the feedback from the patient is not used or does not influence the stimulation data. Also, the stimulation provided by the stimulation unit, inter alia the sequences provided, is/are maintained. This allows a simplified and reliable system. Also, the system may be less complex. It can form an additional system and/or supplement for existing systems or other systems.

It is also possible that the components of the neuromodulation and/or neurostimulation system form partially an open-loop system and partially closed-loop system. For instance, the system may be configured such that the stimulation data may be re-configured and/or adjusted on the basis of data being delivered by the closed-loop system, especially wherein the reconfiguration and/or adjustment is done in real-time. In particular, the closed-loop system and its data may be used to adapt the system to be an open-loop system. Such closed-loop system data may be delivered to the control unit, which then may modify the stimulation data. In other words, the closed-loop system may be used to re-configure and/or adjust the stimulation data of the open-loop system.

Moreover, the control unit is configured such that control may be done in real-time. Real-time may be understood as real-time or close to real-time. For instance, a time frame and short delay between 0.0 to e.g. approximately 50 ms can be understood to fulfill the condition real-time. However, also shorter or longer time delays can be understood to fulfill the condition real-time. In particular, the closed-loop system may work in real-time or may be - in other words - a real-time system such that feedback data e.g. being sensed by the system (more precisely the sensing unit) are processed as input variable for control of the system and that this processing is done in real-time. Overall, this may enable that the stimulation of the patient is adapted immediately to the specific needs and status of the patient. Thus, e.g. loss of conciseness, dizziness, vascular damage as a consequence of hypotension and/or hypertension occurring because the stimulation is not immediately (in real-time) adapted to the patient's blood pressure status may be avoided. In particular, this may be in particular advantageous when the patient is in motion, e.g. changing from lying to sitting and/or standing.

According to a possible embodiment of the system, the system may comprise a CNS stimulation module and a PNS stimulation module. This combination of CNS and PNS stimulation may allow best possible stimulation, which overcomes the drawbacks of providing CNS stimulation or PNS stimulation only.

Moreover, the control unit may be configured such that the PNS stimulation provided by the PNS stimulation module and the CNS stimulation provided by the CNS stimulation module is at least partially interleaved. With this, at least two programming settings may be interleaved, and specific patient requirements may be reflected and/or side effects may be avoided. For example, this feature may allow for shaping of the individualized current fields provided by the CNS stimulation module to fall below the side effect threshold and prevent stimulation of nontargeted anatomical regions and its adjacent structures, thereby reducing side effects and preserving stimulation benefits, and the CNS stimulation provided by the CNS stimulation may be interleaved with PNS stimulation provided to peripheral target zones of the patient, where higher stimulation may be required.

Further, the control unit may be configured such that the PNS stimulation provided by the PNS stimulation module and the CNS stimulation provided by the CNS stimulation module is at least partially superimposed. In particular, this may enable that side effects may be avoided and/or compensated. For instance, this feature may allow for shaping of the individualized current fields provided by the PNS stimulation module to compensate for side effects (of nontargeted anatomical regions and/or its adjacent structures) caused by stimulation provided by the CNS stimulation module. Overall, this may enable preserving stimulation benefits.

Further, the control unit may be capable to independently control and switch on and off either the PNS stimulation module or the CNS stimulation module. It may be possible that switch on and/or off mechanism may be based on physiological data of the patient provide by the sensing unit (feedback information). Alternatively, and/or additionally, a stimulation program may comprise time-regulated PNS and/or CNS stimulation. Overall, this may allow most flexible stimulation of a patient, and further contribute to limit side effects provided the stimulation. Further, this may extend the duration of the energy supply of the implanted pulse generator via a battery, thus reducing costs and effort.

Further, it is conceivable that the neuromodulation and/or neurostimulation system is a first neuromodulation and/or neurostimulation system which may be combined with at least one further neuromodulation and/or neurostimulation system for providing neurostimulation to the patient (e.g. a system providing neurostimulation for restoring motor function of the patient), wherein the control unit 14 of the first neuromodulation and/or neurostimulation system is configured and arranged to trigger that the PNS stimulation provided by the PNS stimulation module and/or the CNS stimulation provided by the CNS stimulation module of the first neuromodulation and/or neurostimulation system is at least partially used for correction of effects of stimulation provided by the at least one further neuromodulation and/or neurostimulation system, e.g. to refine motor output. In particular, this may enable that side effects may be avoided and/or compensated, which are caused by stimulation provided in order to restore motor function. In other words, a patient suffering from SCI (and motor dysfunction and autonomic dysfunction) may be stimulated with a stimulation module in order to restore motor function. This stimulation may cause side effects, in particular side effects on autonomic function (including but not limited to sudden blood pressure elevations/decreases, sudden effect on respiration, uncontrolled bowel and/or bladder sphincter relaxation, uncontrolled sexual function). These side effects may be at least partially avoided and/or limited and/or compensated by stimulation provided with the CNS stimulation module and/or the PNS stimulation module. The stimulation provided with the CNS stimulation module and/or the PNS stimulation module may be superimposed to the stimulation provided with other stimulation module to refine motor output. Otherwise the stimulation provided with the CNS stimulation module and/or the PNS stimulation module and the stimulation provided with other stimulation module to refine motor output may be interleaved.

The system may further comprise at least one of a processor, a telemetry module, a feedback module and/or a further system for providing neuromodulation.

According to the present invention, a method is disclosed, the method characterized in that the method is a method for providing neuromodulation and/or neurostimulation by providing CNS stimulation combined with PNS stimulation, by using a neuromodulation and/or neurostimulation system according to one of claims 1-17.

Further, the present invention relates to the use of a system according to one of claims 1-17 in a method for the treatment of a patient suffering from neurological dysfunction, especially autonomic dysfunction, after spinal cord injury, traumatic brain injury, cerebral palsy, stroke, multiple sclerosis, autonomic failure, autonomic neuropathy and/or cancer of the neurological tissue.

Further details and advantages of the present invention shall now be disclosed in connection with the drawings.

It is shown in
- Fig. 1: a schematical overview of an embodiment of the neuromodulation and/or neurostimulation system 10 according to the present invention, with which the method according to the present invention can be performed; and
- Fig. 2: a schematical overview of a further embodiment of the neuromodulation and/or neurostimulation system 110 according to the present invention, with which the method according to the present invention can be performed.

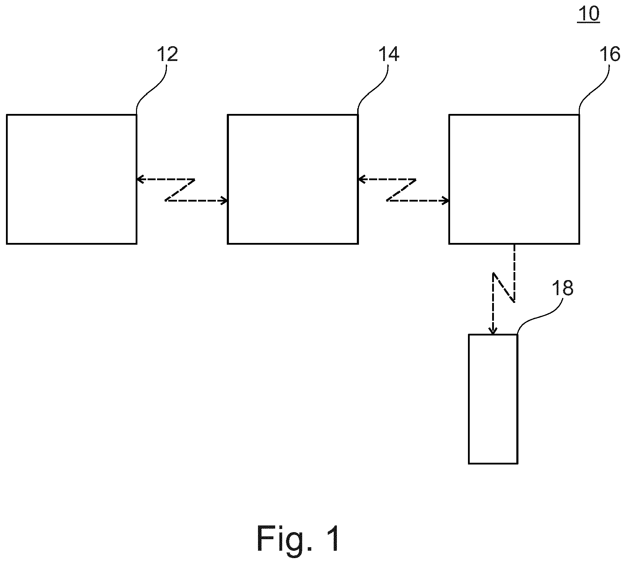

**Fig. 1** shows a schematical overview of an embodiment of the neuromodulation and/or neurostimulation system 10 according to the present invention, with which the method according to the present invention can be performed.

In this embodiment, a neuromodulation and/or neurostimulation system 10 is shown.

The system 10 comprises a sensing unit 12.

In an alternative embodiment, the system 10 can comprise more than one sensing unit 12.

In this embodiment, the sensing unit 12 comprises a blood pressure sensor.

In this embodiment, the sensing unit 12 comprises a finger blood pressure monitoring system.

However, any other type of blood pressure sensor could be generally possible.

In an alternative embodiment, the sensing unit 12 could additionally or alternatively comprise a pulse oximeter, a heart-rate sensor, a breathing rate sensor, a body temperature sensor, a pressure sensor (for instance for sensing bladder internal pressure), a pH sensor, a barometer, an inertial measurement unit, an accelerometer, a gyroscope, a chemical concentration sensor (glucose, water, sodium, calcium), a hormone sensor, a metabolites sensor, a sweat sensor, a gastrointestinal movement sensor, a hydration sensor, an electrolyte sensor, an arterial stiffness sensor, a urine concentration sensor, a urine volume sensor, a bladder volume sensor, a conductivity sensor, a capacitance sensor, a strain gauge sensor, a genital blood flow sensor.

In this embodiment, the system 10 further comprises a control unit 14.

In an alternative embodiment, the system 10 can comprise more than control unit 14.

In this embodiment, the system 10 further comprises a stimulation unit 16.

In an alternative embodiment, the system 10 can comprise more than one stimulation unit 16.

In this embodiment, the stimulation unit 16 is an implantable pulse generator.

In an alternative embodiment, the stimulation unit 16 could be a non-implantable pulse-generator.

It is generally possible that alternatively and/or additionally other components of the system 10 are implantable.

In this embodiment, the system 10 further comprises a CNS stimulation module 18.

In this embodiment, the CNS stimulation module 18 comprises an electrode array for providing CNS stimulation.

In this embodiment, the CNS stimulation module 18 comprises an electrode array for providing EES.

In other words, in this embodiment, the CNS stimulation module comprises an epidural stimulation module capable to provide epidural stimulation, in particular epidural electrical stimulation.

In this embodiment, the electrode array comprises a plurality of electrodes.

In this embodiment, the electrode array comprises 16 electrodes.

However, the electrode array can generally comprise n electrodes, with n being any natural number >0.

In an alternative embodiment, the system 10 could additionally and/or alternatively comprise a PNS stimulation module 20.

The PNS stimulation module 20 could be a Functional Electrical Stimulation (FES) module capable to provide electrical stimulation of peripheral nerves.

In this embodiment, the sensing unit 12 is connected to the control unit 14.

This connection is a direct connection.

Further, this connection is a bidirectional connection.

Further, this connection is a wireless connection.

However, alternatively, the connection between the sensing unit 12 and the control unit 14 could be an indirect and/or unidirectional and/or cable-bound connection.

In this embodiment, the control unit 14 is connected to the stimulation unit 16.

This connection is a direct connection.

Further, this connection is a bidirectional connection.

Further, this connection is a wireless connection.

However, alternatively, the connection between the control unit 14 and the stimulation unit 16 could be an indirect and/or unidirectional and/or cable-bound connection.

In this embodiment, the stimulation unit 16 is connected to the CNS stimulation module 18.

This connection is a direct connection.

Further, this connection is a unidirectional connection.

Further, this connection is a cable-bound connection.

However, alternatively, the connection between the stimulation unit 16 and the CNS stimulation module 18 could be an indirect and/or bidirectional and/or wireless connection.

In this embodiment, the sensing unit 12 provides a sensor signal correlating with blood pressure of a patient equipped with the system 10.

In this embodiment, the sensing unit 12 provides a sensor signal indicating systolic blood pressure of the patient equipped with the system 10.

In general, the sensing unit 12 can provide a sensor signal correlating with a physiological value, which describes neurological function or dysfunction, preferably autonomic function or dysfunction of a patient.

In this embodiment, the sensor signal is provided to the control unit 14.

In this embodiment, the control unit 14 detects hypotension of the patient based on the sensor signal.

Further, in this embodiment, the control unit 14 triggers neurostimulation for stabilization of blood pressure of the patient.

In general, the control unit 14 can detect a dysfunction of neurological function, preferably autonomic function of the patient based on the sensor signal and to trigger neuromodulation for stabilization and/or treatment of the neurological function, preferably autonomic function of the patient.

In this embodiment, the control unit 14 does the control in real-time.

In other words, the neuromodulation provided by the CNS stimulation module 18 is in real-time adapted to the sensor signal provided by the sensing unit 12.

In this embodiment, the components of the neuromodulation and/or neurostimulation system 10 form a closed-loop system 10.

In an alternative embodiment, the components of the neuromodulation and/or neurostimulation system 10 can form an open-loop system 10.

In a further alternative system 10, the components of the neuromodulation and/or neurostimulation system 10 can form partially an open-loop system 10 and partially a closed-loop system 10.

Not shown in Fig. 1 is that alternatively, and/or additionally to the epidural stimulation module, the CNS stimulation module 18 could comprise a transcutaneous stimulation module capable to provide transcutaneous stimulation and/or a subdural stimulation module capable to provide subdural stimulation and/or an intracortical stimulation module capable to provide intracortical stimulation and/or an intraspinal stimulation module capable to provide intraspinal stimulation.

Further not shown in Fig. 1 is that the sensing unit 12 could alternatively and/or additionally comprise an electrocardiogram (ECG).

Further not shown in Fig. 1 is that the sensing unit 12 could alternatively and/or additionally comprise an electroencephalogram (EEG) and/or an electromyogram (EMG), a patch clamp, a voltage clamp, an extracellular single-unit recorder and/or a recorder of local field potentials. Further not shown in Fig. 1 is that the sensing unit 12 could additionally and/or alternatively be or comprise at least one trigger, e.g. a manual trigger, to be triggered by the patient and providing a signal describing dysfunction of the autonomic function, e.g. blood pressure, of the patient.

Further not shown in Fig. 1 is that the sensing unit 12 and/or the control unit 14 can optimize stimulation parameters for stabilization and/or treatment of the neurological function, preferably autonomic function of the patient.

In general, the system 10 can be used in a method for the treatment of a patient suffering from neurological dysfunction, especially autonomic dysfunction, after spinal cord injury, traumatic brain injury, cerebral palsy, stroke, multiple sclerosis, autonomic failure, autonomic neuropathy and/or cancer of the neurological tissue, and/or any other disease impairing the nervous system of the patient.

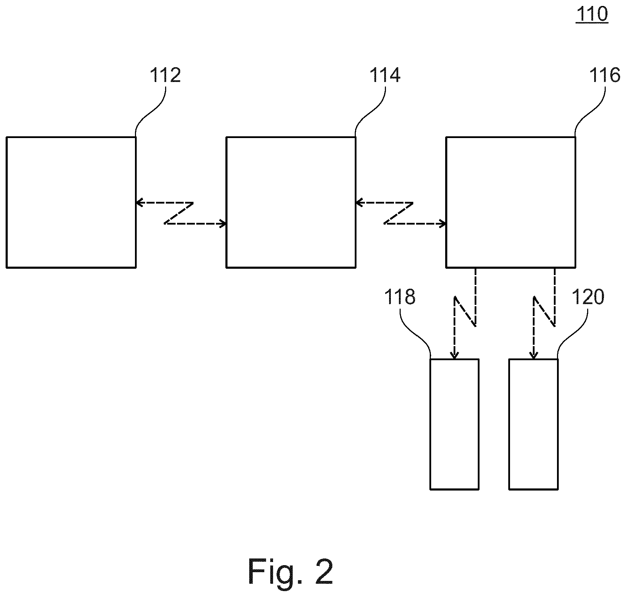

**Fig. 2** shows a schematical overview of a further embodiment of the neuromodulation and/or neurostimulation system 110 according to the present invention, with which the method according to the present invention can be performed.

The system 110 comprises the structural and functional features of the system 10 disclosed in Fig. 1. The corresponding reference numbers in Fig. 2 for identical or similar elements or features correspond to the corresponding reference numbers of Fig. 1 and to reflect this, then these reference numbers are taken and increased by the value 100.

In addition to the CNS stimulation module 118 the system 110 comprises a PNS stimulation module 120.

In this embodiment, the stimulation unit 116 is connected to the CNS stimulation module 118 and the PNS stimulation module 120.

Both connections are direct connections.

Further, both connections are unidirectional connections.

However, alternatively, the connection between the stimulation unit 116 and the CNS stimulation module 118 and/or the stimulation unit 116 and/or the PNS stimulation module 120 could be an indirect and/or bidirectional connection.

The connection between the stimulation unit 116 and the CNS stimulation module 118 is a cable-bound connection.

However, in an alternative embodiment, this connection could be a wireless connection.

The connection between the stimulation unit 116 and the PNS stimulation module 120 is a wireless connection.

However, in an alternative embodiment, this connection could be a cable-bound connection.

In this embodiment, the CNS stimulation module 118 and the PNS stimulation module 120 are indirectly connected (via the stimulation unit 116).

Not shown in Fig. 2 is that the CNS stimulation module 118 and the PNS stimulation module 120 could be directly connected (wireless or cable-bound connection, unidirectional or bidirectional connection).

In this embodiment the CNS stimulation module 118 is an epidural stimulation module for providing EES.

In this embodiment the CNS stimulation module 118 comprises an electrode array, which is implantable.

In this embodiment, the PNS stimulation module 120 is a Functional Electrical Stimulation (FES) module capable to provide electrical stimulation to peripheral nerves.

In an alternative embodiment, the PNS stimulation module 120 provide electrical stimulation to smooth muscles of blood vessels.

In this embodiment, the FES module provides stimulation transcutaneously.

In this embodiment, the FES module comprises an electrode placed transcutaneously and providing electrical stimulation to the lower legs of a patient.

Alternatively, the FES module could be implantable and/or implanted (electrodes implanted).

In general, the FES module could comprise a plurality of electrodes.

In general, the CNS stimulation module 118 can be at least partially implantable or at least partially implanted and/or that the PNS stimulation module 120 can be at least partially implantable or at least partially implanted.

In this embodiment, the control unit 114 controls the stimulation provided by the PNS stimulation module 120 and the CNS stimulation module 118 such that the stimulation provided by the two stimulation modules 118, 120 is interleaved.

In other words, the control unit 114 is configured such that the PNS stimulation provided by the PNS stimulation module 120 and the CNS stimulation provided by the CNS stimulation module 118 are least partially interleaved.

Alternatively, the control unit 114 can be configured such that the PNS stimulation provided by the PNS stimulation module 120 and the CNS stimulation provided by the CNS stimulation module 118 is at least partially superimposed.

In general, the system 110 can be used for a method for providing neuromodulation and/or neurostimulation by providing CNS stimulation combined with PNS stimulation.

Not shown in Fig. 2 is that it is generally possible that the control unit 114 can independently control and switch on and off either the PNS stimulation module 120 or the CNS stimulation module 118.

Further not shown in Fig. 2 is that the neuromodulation and/or neurostimulation system 110 can be a first neuromodulation and/or neurostimulation system 110 which is configured and arranged to be combined with at least one further neuromodulation and/or neurostimulation system for providing neurostimulation to the patient, wherein the control unit 114 of the first neuromodulation and/or neurostimulation system 110 is configured and arranged to trigger that the PNS stimulation provided by the PNS stimulation module 120 and/or the CNS stimulation provided by the CNS stimulation module 118 of the first neuromodulation and/or neurostimulation system 110 is at least partially used for correction of effects of stimulation provided by the at least one further neuromodulation and/or neurostimulation system.

### References

- 10, 110: system
- 12, 112: sensor unit
- 14, 114: control unit
- 16, 116: stimulation unit
- 18, 118: CNS stimulation module
- 20, 120: PNS stimulation module

## Claims

1. A neuromodulation and/or neurostimulation system (10, 110) comprising at least the following components:
- at least one sensing unit (12, 112), wherein the sensing unit (12, 112) is configured to provide a sensor signal correlating with a physiological value, which describes neurological function or dysfunction, preferably autonomic function or dysfunction of a patient;
- at least one control unit (14, 114),
- at least one stimulation unit (16, 116),
- at least one Central Nervous System (CNS) stimulation module for providing CNS stimulation (18, 118), and/or at least one Peripheral Nervous System (PNS) stimulation module for providing PNS stimulation (20, 120);
wherein the control unit (14, 114) is configured to detect a dysfunction of the neurological function, preferably autonomic function of the patient based on the sensor signal and to trigger neuromodulation for stabilization and/or treatment of the neurological function, preferably autonomic function of the patient.

2. The system (10, 110) according to claim 1, **characterized in that** at least one of the components of the neuromodulation and/or neurostimulation system (10) is implantable.

3. The system (10, 110) according to claim 1 or claim 2, **characterized in that** the sensing unit (12, 112) is or comprises at least one of an electrocardiogram (ECG), an electroencephalogram (EEG), or an electromyogram (EMG), a patch clamp, a voltage clamp, an extracellular single-unit recorder, a recorder of local field potentials.

4. The system (10, 110) according to one of the preceding claims, **characterized in that** the sensing unit (12, 112) is or comprises at least one of a blood pressure sensor, a pulse oximeter, a heart-rate sensor, a breathing rate sensor, a body temperature sensor, a pressure sensor, a pH sensor, a barometer, an inertial measurement unit, an accelerometer, a gyroscope, a chemical concentration sensor (glucose, water, sodium, calcium), a hormone sensor, a metabolites sensor, a sweat sensor, a gastrointestinal movement sensor, a hydration sensor, an electrolyte sensor, an arterial stiffness sensor, a urine concentration sensor, a urine volume sensor, a bladder volume sensor, a conductivity sensor, a capacitance sensor, a strain gauge sensor, a genital blood flow sensor.

5. The system (10, 110) according to one of the preceding claims, **characterized in that** the sensing unit (12, 112) is configured and arranged and/or the control unit (14, 114) is configured and arranged for optimizing stimulation parameters for stabilization and/or treatment of the neurological function, preferably autonomic function of the patient.

6. The system (10, 110) according to one of the preceding claims, **characterized in that** the sensing unit (12, 112) is or comprises at least one trigger, especially a manual trigger, to be triggered by the patient and providing a signal describing dysfunction of the autonomic function of the patient.

7. The system (10, 110) according to one of the preceding claims, **characterized in that** the Central Nervous System (CNS) stimulation module (18, 118) is or comprises an epidural stimulation module capable to provide epidural stimulation and/or a trans-cutaneous stimulation module capable to provide trans-cutaneous stimulation and/or a subdural stimulation module capable to provide subdural stimulation and/or an intracortical stimulation module capable to provide intracortical stimulation and/or an intraspinal stimulation module capable to provide intraspinal stimulation.

8. The system (10, 110) according to one of the preceding claims, **characterized in that** the Peripheral Nervous System (PNS) stimulation module (20, 120) is a Functional Electrical Stimulation (FES) module capable to provide electrical stimulation of peripheral nerves.

9. The system (10, 110) according to one of the preceding claims, **characterized in that** the Central Nervous System (CNS) stimulation module (18, 118) is at least partially implantable or at least partially implanted and/or that the Peripheral Nervous System (PNS) stimulation module (20, 120) is at least partially implantable or at least partially implanted.

10. The system (10, 110) according to one of the preceding claims, **characterized in that** the components of the neuromodulation and/or neurostimulation system (10, 110) form a closed-loop system.

11. The system (10, 110) according to one of the preceding claims, **characterized in that** the components of the neuromodulation and/or neurostimulation system (10, 110) form an open-loop system.

12. The system (10, 110) according to claim 9 and claim 10, **characterized in that** the components of the neuromodulation and/or neurostimulation system form partially an open-loop system and partially closed-loop system.

13. The system (10) according to one of the preceding claims, **characterized in that** the control unit (14, 114) is configured such that control is done in real-time.

14. The system (10, 110) according to one of the preceding claims, **characterized in that** the control unit (14, 114) is configured such that the PNS stimulation provided by the PNS stimulation module (20, 120) and the CNS stimulation provided by the CNS stimulation module (18, 118) is at least partially interleaved.

15. The system (10, 110) according to one of the preceding claims, **characterized in that** the control unit (14, 114) is configured such that the PNS stimulation provided by the PNS stimulation module (20, 120) and the CNS stimulation provided by the CNS stimulation module (18, 118) is at least partially superimposed.

16. The system (10, 110) according to one of the preceding claims, **characterized in that** the control unit (14, 114) is capable to independently control and switch on and off either the PNS stimulation module (20, 120) or the CNS stimulation module (18, 118).

17. The system (10, 110) according to one of the preceding claims, **characterized in that** the system (10, 110) is a first neuromodulation and/or neurostimulation system (10, 110) which is configured and arranged to be combined with at least one further neuromodulation and/or neurostimulation system for providing neurostimulation to the patient, wherein the control unit (14, 114) of the first neuromodulation and/or neurostimulation system (10, 110) is configured and arranged to trigger that the PNS stimulation provided by the PNS stimulation module (20, 120) and/or the CNS stimulation provided by the CNS stimulation module (18, 118) of the first neuromodulation and/or neurostimulation system (10, 110) is at least partially used for correction of effects of stimulation provided by the at least one further neuromodulation and/or neurostimulation system.

18. A method for providing neuromodulation and/or neurostimulation by providing Central Nervous System (CNS) stimulation combined with Peripheral Nervous System (PNS) stimulation, by using a neuromodulation and/or neurostimulation system (10, 110) according to one of the preceding claims.

19. The use of a system (10, 110) according to any of claims 1-17 in a method for the treatment of a patient suffering from neurological dysfunction, especially autonomic dysfunction, after spinal cord injury, traumatic brain injury, cerebral palsy, stroke, multiple sclerosis, autonomic failure, autonomic neuropathy and/or cancer of the neurological tissue.
